Europäisches Patentamt

European Patent Office

Office européen des brevets

(19)

(11) Publication number: **0 326 628**
**A1**

(12) **EUROPEAN PATENT APPLICATION**

(21) Application number: **88101402.1**

(22) Date of filing: **01.02.88**

(51) Int. Cl.4: **A61K 31/47**

(43) Date of publication of application:
**09.08.89 Bulletin 89/32**

(84) Designated Contracting States:
**BE DE FR GB IT NL SE**

(71) Applicant: **VIPONT PHARMACEUTICAL, INC.**
**P.O. Box 460**
**Fort Collins Colorado 80522(US)**

(72) Inventor: **Sakamoto, Seizaburo**
**17 Crafts Road**
**Chestnut Hill Massachusetts 02167(US)**
Inventor: **Sugiyama, Katsumi**
**Apt 21B 400 Brookline Avenue**
**Boston Massachusetts 02115(US)**

(74) Representative: **Vossius & Partner**
**Siebertstrasse 4 P.O. Box 86 07 67**
**D-8000 München 86(DE)**

(54) **Method for inhibiting the release of histamine.**

(57) The present invention deals with the inhibition of histamine release from mammalian mast cells by contacting such cells with sanguinarine, a benzophenanthridine alkaloid.

EP 0 326 628 A1

## METHOD FOR INHIBITING THE RELEASE OF HISTAMINE

The present invention relates to a method for controlling the release of histamine from mast cells

Benzo-c-phenanthridine alkaloids can be extracted from plants of the families Papaversease, Fumariaceae, and Berberidaceae. Some of the plants of these families includes Sanguinaria canadensis, Macleaya cordata, Bocconia frutescens, Carydalis sevctcozii, C. ledebouni, Argemone mexicanus, and Chelidonium majus. Among the most important benzo-c-phenanthridine alkaloids obtained from these plants are sanguinarine, chelirubine, macarpine, allocryptopine, protopine, hemochelidonene, sanguilatine, sanguirubine, and chelerythrine.

The best known of these alkaloids is sanguinarine, which has been extracted from the Sanguinaria canadensis plant (otherwise known as bloodroot, teteroot, redroot, puccoon, etc.) a perennial herb native to North America. The sanguinaria plant and its juices have been used for various proposes in pre-historic and historic times. The plants have been used, in particular, as a folk remedies. The plants have generally been used while, either undried (fresh) or dried, and the usual procedure is to powder the dried plant and mix it with a carrier. This folk remedy has been tried for such conditions as bronchitis, dysentery, ringworm and a substantial list of other ailments.

The pure chemicals sanguinarine, chelerythrine, protopine, chelerubine, berberine, chelilutine, sanguilatine, macarpine, sanguirubine and allocryptopine can be isolated from plants other than Sanguinaria. They are also available, although rarely, from some chemical supply houses. Semi-purified forms of the alkaloids are commercially available, and these are generally referred to as sanguinarine nitrate and sanguinarine sulfate. These compounds are the salts of the mixed alkaloids of the plant Sanguinaria: mainly sanguinarine, chelerythrine, and protopine. While few references can be found in the literature regarding the usage of any of the pure benzophenanthridine alkaloids, plants containing such compounds have been used for a wide variety of medical ailments.

The alkaloid sanguinarine in solution has been shown to have some antifungal and antiprotozoan properties. The sanguinarine is applied as an emulsion topically to fungal infections. The antibacterial activity of sanguinarine has been found to vary with attached radicals, and various salts of sanguinarine have been shown to have some activity. The hydrochloride and the sulfate salts have been found to have some activity against certain bacteria at certain concentrations. Sanguinarine nitrate is reported to have some bacteriostatic action against various types of bacteria.

The use of an extract of Sanguinaria canadensis as an ingredient in oral cleaning preparation, in particular, toothpaste, is disclosed in U.S. Patent No. 4,145,412.

The extract may be produced by treating a finely cut or ground bloodroot with an organic solvent, such as methanol, acidulated methanol, acidulated ethanol, acidulated methanol-water solutions, acidulated ethanol-water solutions, and mixtures thereof.

The bloodroot is thoroughly stirred with several volumes of the solvent, and is maintained in the solvent for a prolonged time (e.g. 24 hours or more, at a temperature of about 60°C.). In accordance with one technique the solution can be filtered and the solvent evaporated. The residue can then be dissolved (e.g. in chloroform),treated with concentrated hydrochloric acid, filtered and then dried. The dried extract may be taken up in warm glycerine (65°C.) for mixing with a carrier. In accordance with other techniques, after the plant has been extracted with solvent, (a) the extract can be precipitated with an acid salt which is soluble in the solvent, (b) the precipitated salt is redissolved in water, (c) acid is added to form a precipitate, and (d) the precipitate is collected.

The present invention therefor deals with the inhibition or suppression of histamine release from mammalian mast cells by contacting such cells with sanguinarine, a benzophenanthridine alkaloid. In giving evidence of the invention it has been discovered that Sanguinarine (1-10µ M) inhibited histamine release stimulated by compound 48/80, a well known histamine release agent. This inhibitory action was also found when histamine release was induced by other known histamine releasers, such as concanavalin A, histidine-rich polypetide (Fraction-A), and calcium-ionophore A 23187, while this was not the case with a well known surfactant Triton X-100 (octylphenoxy polyethryethanol). It is believed that the sanguinarine acts as a stabilizer of the membrane of the mast cells.

Sanguinarine heretofore stated is a benzophenanthridine alkaloid extracted, for example, from the rhizomes of the plantSanguinaria candensis; as seen in American Pharmaceutical Association, National Formulary, 7th Revision (1940) pp. 191, 368 and 416. It has been shown that sanguinarine exhibits various biological activities such as antimicrobial activity; as seen in Johnson, C.C. Johnson, G. and Poe, C.F. (1952) Acta Pharmacol. Toxicol. 8, 71-78; and Dzink, J.L. and Socransky, S.S. (1985) Antimicrobial Agents and Chemother. 27, 663-665. There is evidence that it also inhibits Na, K-ATPase, as seen in Straub, K.D.

and Carver, P. (1970) Biochem. Biophys. Rec. Commun. 62, 913 and 922. It has also shown cation-transport, as seen in Cala, P.M., Norby, J.G. and Toteson, D.C. (1982) J. Membrane Biol. 64, 23-31. It has been shown to inhibit yeast respiration, as seen in Vallejos, R.H. and Roveri, O.A. (1972) Biochem. Pharmacol. 21, 2179-3182. More recently it has also been found that sanguinarine has antiplaque activity as seen in Southard, G.L., Boulware, R.T., Walborn, D.R., Groznik, W.J., Throne, E.E. and Yankell, S.L. (1981) . Am. Dent. Assoc. 108, 338-341. Sanguinarine also has anti-inflammatory activity as demonstrated in Lenfeld, J., Kroutil, M., Marsalek, E., Slavik, J., Preininger, V. and Simanak, V. (1981) Planta Med. 43, 161-165. Finaly as perhaps the latest finding it is now known that sanguinarine demonstrates significant inhibition of bone resorption in vitro, as seen in Cowen, K.D., Sakamoto, S., Sakamoto, M. and Southard, G.L. (1986) J. Dent. Res. 65, 246.

In exemplifying the invention, mast cells were isolated from the peritoneal fluid of male Wistar rat as described in Sugiyama, K. (1971) Japan J. Pharmacol. 21, 209-226. The mast cells were suspended in a phosphate buffered saline solution consisting of 154 mM sodium chloride, 2.7mM potassium chloride, 0.9 mM calcium chloride, 6.7 mM $KH_2PO_4$-$Na_2HPO_4$, (pH 7.3), and 0.01% bovine serum albumin obtained from Sigma.

For the determination of histamine release and its inhibition by sangainarine., mast cells at a cell density of $10^5$ cells/ml suspended in 0.5 ml of phosphate-buffered saline, were incubated at 37°C for 10 minutes with histamine releasers, more about which is stated below. Sanguinarine was added 5 minutes before the specific histamine releaser. After the action was terminated by the addition of 1.5 ml of ice-cold phosphate-buffered saline solution, the samples were centrifuged at 2,000 rpm for 15 minutes. The histamine in the supernatants and precipitates was determined according to the method of Shore, P.A., Burkhalter, A. and Cohn N.H. (1959) J. Pharmacol. Exp Ther. 127, 182-186. Release of histamine was expressed as a percentage of the total cell content. Percentages of inhibition by sanguinarine were measured according to the following equation:

$$\text{Inhibition \%} = \left(1 - \frac{\text{Histamine released from mast cells (with Sanguinarine)}}{\text{Histamine released from mast cells (without Sanguinarine}}\right) X$$

The sanguinarine chloride was provided from Vipont Laboratories Inc. and is manufactured in the manner disclosed above.

Histidine-rich polypetide fraction A, known as HRP(F-A) was purified from human saliva in accordance with the procedure of Sugiyama, K., Suzuki, Y and Furuta, H (1985) Life Sciences. 37, 475-480. The comparison was carried out with known histamine releasers, namely, 48/80; Concanavalin A; calcium-ionophore A23187. These releasers may be obtained from Sigma Co.

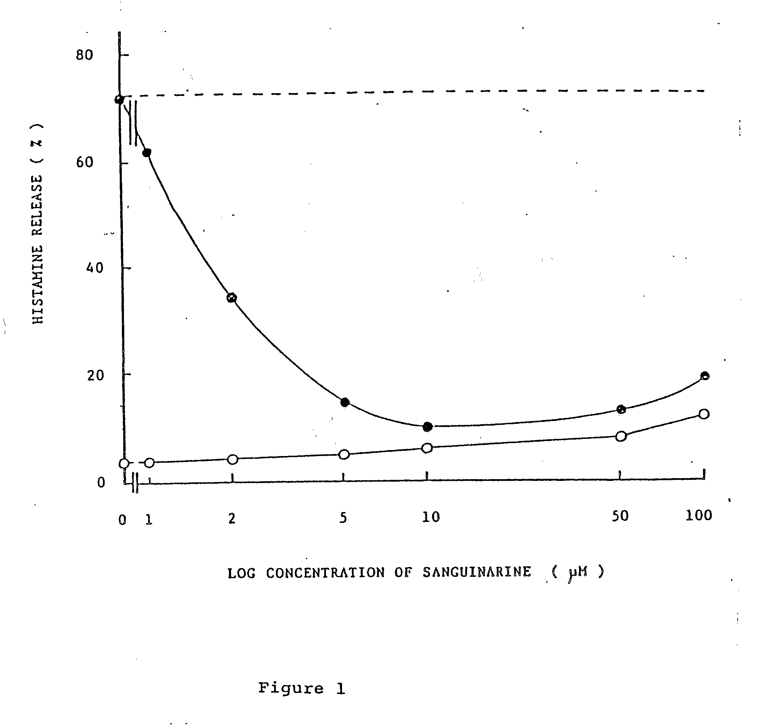

In the results it was noted that 48/80 induced histamine release from mast cells was inhibited by sanguinarine in a dose-dependent manner as can be seen from Figure 1; wherein the $IC_{50}$ (inhibition concentration) was about 2 μ M. Histamine release induced by sanguinarine itself was not apparent in a concentration less than 100μ M. The inhibitory effect of sanguinarine was also observed in the histamine release stimulated by Concanavalin A, histidine-rich polypeptide and Ca-ionophore A 23187, but not in the histamine release stimulated by Triton X-100 (Table 1).

It has been recognized that histamine release stimulated by compound 48/80 from mast cells is initiated by the fusion of plasma and granule membrane (exocytosis) as discussed by Uvnas, B. (1974) Fed. Proc. 33, 2172-2176. Concanavalin A. stimulates histamine release from mast cells in the presence of phosphatidylserine and calcium ions, discussed by Sugiyama, K., Sasaki, J. and Yamasaki, H. (1975) Japan. J. Pharmacol. 25, 485-487. Calcium-ionophore A 23187 also releases histamines dependent upon the external Calcium ions. On the other hand, histamine release evoked by compound 48/80 and histidine-rich polypeptide is independent of extracellular calcium. Sanguinarine markedly inhibited histamine release induced by a variety of different agents except Triton X-100 (a detergent). These observations suggest that sanguinarine may not inhibit a calcium-dependent mechanism required for the initiation of histamine release.

It has been reported in Pearce, F.L., Atkinson, G., Ennis, M., Trench, A., Weston, P.M. and White, J.R. (1979) in "The Mast Cell "(Pepys, J. and Edwards, A.M., ed.) pp 69-75, Pitman Medical Publishing Co. Ltd, England that anti-allergy drugs such as theophylline and cromoglycate inhibited the histamine release produced by compound 48/80, calcium-ionophore A23187, and basic peptide 401, (a bee venom); the latter mentioned in the immediately forgoing literature article. They indicated that the $IC_{50}$ of these drugs was in a 0.01-1mM concentration range. These values are about 10 to 100 times less active than sanguinarine inhibited histamine release. It has also been shown in Yamasaki, H. and Saeki K. (1967) Arch, int. Pharmacodyn. 168, 166-179 that compound 48/80 induced histamine release was inhibited by the anti-inflammatory agents, indomethacin, sodium salicylate, and hydrocortisone. The $IC_{50}$ for those agents was 75 $\mu$ M, 1.6mM and 2.1 mM, respectively. Thus, in comparison with the $IC_{50}$ of these drugs, sanguinarine inhibits histamine release at very low concentrations.

It is pointed out that the mechanisms by which sanguinarine inhibits histamine release are at present unknown. It is speculated that sanguinarine acts as a stabilizer of membrane activities relating to the physical chemical nature of exocytosis involving transfer of the inner granules to the outside.

TABLE 1

| Inhibition by Sanguinarine of Histamine Release from Rat Isolated Mast Cells | | | |
|---|---|---|---|
| Releasing agents (Conc.) | Sanguinarine ($\mu$M) | Histamine Release (%) | % Inhibition |
| Compound 48/80 (0.5 $\mu$ g/ml) | - 5 | 72.3 15.4 | 78.7 |
| Con A * (10 $\mu$g/ml) | - 5 | 62.9 27.3 | 56.6 |
| A23187 (1 $\mu$ M) | - 5 | 57.7 21.1 | 63.4 |
| HRP (16 $\mu$ M) | - 5 | 57.6 17.6 | 69.4 |
| Triton X-100 (0.01 %) | - 5 | 84.8 93.8 | -10.6 |

*in the presence of phosphatidylserine (10 $\mu$ g/ml)

With regard to Figure 1, inhibition by sanguinarine of histamine release induced by compound 48/80 is shown. The mast cells were incubated at 37° C for 10 minutes with and without compound 48/80 in the presence of sanguinarine in varying concentrations. (●) Sanguinarine with compound 48/80 (0.5 $\mu$ g/ml), (0) Sanguinarine without compound 48/80.

## Claims

1. The use of a benzo-c-phenanthridine alkaloid for preparing a pharmaceutical composition for inhibiting the release of histamine from mammalian mast cells.

2. The use according to claim 1 wherein the benzo-c-phenanthridine alkaloid is sanguinarine.

3. The use according to Claim 2 wherein the sanguinarine is in a diluent.

4. The use according to any one of Claims 1 to 3 wherein the release of histamine from mammalian mast cells is induced and said release is then controlled by contacting said mast cells with benzo-c-phenanthridine alkaloid.

Figure 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | US-A-4 737 503 (SAKAMOTO)<br>* Whole document * | 1-4 | A 61 K 31/47 |
| X | THERAPIE, vol. 28, no. 4, 1973, pages 767-774, FR; A.H. DIL: "Activité anti-histaminique de la fumarine"<br>* Abstract; pages 773-774: "Discussion" * | 1-4 | |
| X | ACTA PHARMACEUTICA HUNGARICA, vol. 39, 1969, pages 247-251; K. BARNA: "Antitussive action of isoquinoline derivatives"<br>* Page 250, table III; page 251, abstract * | 1-4 | |
| A | JOURNAL OF POSTGRADUATE MEDICINE, vol. 26, no. 1, 1980, pages 28-33; A.B. VAIDYA et al.: "Inhibition of human pregnancy plasma diamine oxidase with alkaloids of Argemone mexicana – berberine and sanguinmarine"<br>* Abstract; pages 31-32: "Discussion" * | 1-4 | TECHNICAL FIELDS SEARCHED (Int. Cl.4)<br><br>A 61 K 31/00 |

The present search report has been drawn up for all claims

| Place of search | Date of completion of the search | Examiner |
|---|---|---|
| THE HAGUE | 20-09-1988 | ISERT B. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P0401)